# EUROPEAN PATENT APPLICATION

(11) **EP 4 809 973 A1**
(43) Date of publication of application: **23.09.2026**
(21) Application number: 24891761.9
(22) Date of filing: 13.11.2024
(51) Int. Cl.: A61C 8/00, A61C 1/08, A61B 17/88, B25B 17/00

(54) **DENTAL IMPLANT DRIVER**

(30) Priority: 14.11.2023 KR 20230156910
(71) Applicant: Osstemimplant Co., Ltd., Gangseo-gu Seoul 07789 (KR)
(72) Inventor: LEE, Young Seok, Seoul 07789 (KR); HEO, Jae Chan, Seoul 07789 (KR)
(74) Representative: Germain Maureau
(86) International application number: PCT/KR2024/017905
(87) International publication number: WO 2025/105815

(57) **Abstract**

An embodiment ofthe present invention provides a dental implant driver for aligning the planting direction of an implant planted in the alveolar bone, the dental implant driver comprising: a polygonal insert part that is provided at one end and can be inserted so as to fit into a polygonal groove of the implant; a first direction identification part located above the insert part and having at least one identification element which is aligned with the direction of at least one surface of the insert part so as to align the planting direction of the implant; and a second direction identification part located above the first direction identification part and having an angle or surface which is aligned with the direction of the identification element so as to assist in aligning the planting direction of the implant.

## Description

### [Technical Field]

The present invention relates to a dental implant driver.

### [Background Art]

An implant originally means a substitute for restoring human tissue when the human tissue has been lost, but in the dental field, it refers to an artificially made tooth root. A dental implant procedure is performed through a process of implanting, into an alveolar bone, an implant made of titanium or the like that does not cause a rejection reaction in the human body so as to replace the root of a lost tooth, causing the implant to osseointegrate, and coupling an abutment and a prosthesis to the implant.

In general, coupling between an implant and an abutment is achieved by inserting and coupling a polygonal protrusion of the abutment, which is formed in a hexagonal shape, into a polygonal groove of the implant. At this time, in order for the abutment and the prosthesis to be coupled to the implant in a desired direction, the implant needs to be implanted in the alveolar bone with the polygonal groove of the implant aligned in a specific direction. In other words, in order for the abutment and the prosthesis to be aligned in a desired direction, the implant first needs to be aligned in a specific direction in the alveolar bone.

Meanwhile, after implanting the implant in the alveolar bone, a practitioner finely adjusts the direction of the implant by using a dental implant driver. However, since the implant is in a state of being implanted in the alveolar bone, it is difficult to accurately adjust the implant in a desired direction. Accordingly, the practitioner needs to determine the direction of the implant through the relative positional relationship between the dental implant driver and a guide template. However, a conventional dental implant driver does not provide a function for checking the implantation direction of the implant or checking the relative positional relationship with the guide template. Accordingly, when the practitioner aligns the implant with a conventional dental implant driver, there has been a difficulty in accurately aligning the implant in a desired direction.

### Prior Art Document

Korean Registered Patent Publication No. 10-1598495 (Feb. 23, 2016)

### [Disclosure]

### [Technical Problem]

The present invention has been made to solve the above-described problems of the prior art, and an object of the present invention is to provide a dental implant driver capable of easily determining an implantation direction of an implant implanted in an alveolar bone during an implant procedure and aligning the implant in a desired direction.

### [Technical Solution]

In order to achieve the above object, one aspect of the present invention provides a dental implant driver for aligning an implantation direction of an implant implanted in an alveolar bone, the dental implant driver comprising: a polygonal insertion portion provided at one end and configured to be inserted into and fitted to a polygonal groove portion of the implant; a first direction recognition portion located above the insertion portion and having formed thereon one or more identification elements having a direction aligned with at least one face of the insertion portion for aligning the implantation direction of the implant; and a second direction recognition portion located above the first direction recognition portion and having formed thereon an angle or a face having a direction aligned with the identification element for assisting in aligning the implantation direction of the implant.

According to one embodiment of the present invention, the one or more identification elements may be formed spaced apart from one another at equal intervals along a circumference of the first direction recognition portion.

According to one embodiment of the present invention, the one or more identification elements may be formed in the same number as the number of faces of the insertion portion.

According to one embodiment of the present invention, the one or more identification elements may be formed in a recessed groove shape.

According to one embodiment of the present invention, the one or more identification elements may be color-treated to facilitate identification.

According to one embodiment of the present invention, the second direction recognition portion may be formed in an equilateral triangular shape.

According to one embodiment of the present invention, the second direction recognition portion may be formed in a hexagonal shape.

According to one embodiment of the present invention, a direction recognition groove may be formed in at least one face of the second direction recognition portion.

According to one embodiment of the present invention, the second direction recognition portion may be formed in a fan shape.

According to one embodiment of the present invention, the second direction recognition portion may be formed with a blade protruding toward one side in a radial direction.

According to one embodiment of the present invention, the second direction recognition portion may be formed with two blades protruding toward both sides in a radial direction.

### [Advantageous Effects]

According to one aspect of the present invention, since the dental implant driver of the present invention includes the first direction recognition portion and the second direction recognition portion, it is possible to easily determine the implantation direction of the implant implanted in the alveolar bone and intuitively align the implant in a desired direction, even in a situation in which it is difficult to secure a field of view during an implant procedure.

The effects of the present invention are not limited to the effects described above, and it should be understood that the present invention includes all effects that can be inferred from the configuration of the invention described in the detailed description of the invention or in the claims.

### [Description of Drawings]

FIG. 1 illustrates a view showing a state in which the dental implant driver according to an embodiment of the present invention is used.
FIG. 2 is a perspective view of the dental implant driver according to an embodiment of the present invention.
FIG. 3 is a view showing the dental implant driver according to an embodiment of the present invention and an implant arranged together.
FIGS. 4A and 4B are a perspective view and a plan view, respectively, showing a state before alignment of the direction of an implant using the dental implant driver according to an embodiment of the present invention.
FIGS. 5A and 5B are a perspective view and a plan view, respectively, showing a state after alignment of the direction of an implant using the dental implant driver according to an embodiment of the present invention.
FIGS. 6 to 9 are views showing various embodiments of the dental implant driver of the present invention.

### [Modes of the Invention]

Hereinafter, the present invention will be described with reference to the accompanying drawings. However, the present invention may be embodied in various different forms, and thus is not limited to the embodiments described herein. In order to clearly describe the present invention in the drawings, portions unrelated to the description are omitted, and like reference numerals are assigned to like portions throughout the specification.

Throughout the specification, when one portion is described as being "connected" to another portion, this includes not only a case in which the one portion is "directly connected" to the other portion, but also a case in which the one portion is "indirectly connected" to the other portion with another member interposed therebetween. Further, when one portion is described as "including" a certain component, this means that the one portion may further include another component, rather than excluding another component, unless specifically stated otherwise.

Hereinafter, embodiments of the present invention will be described in detail with reference to the accompanying drawings.

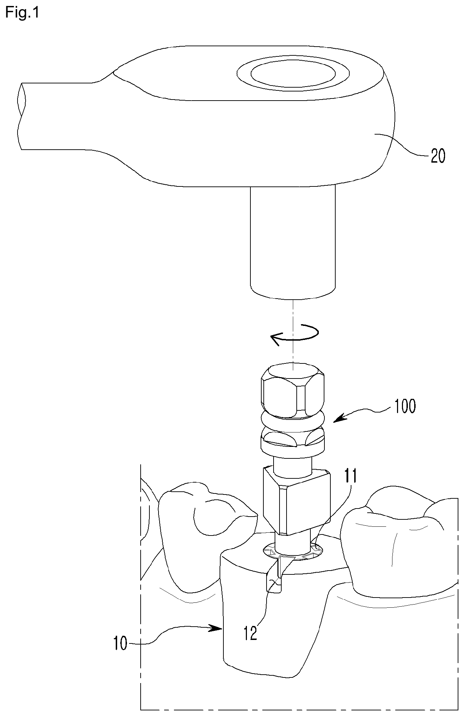

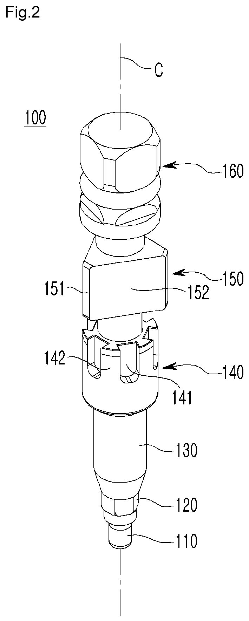

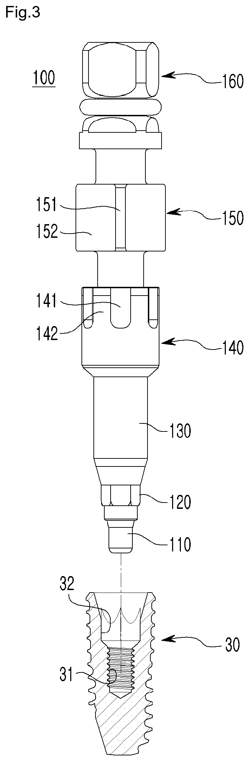

FIG. 1 illustrates a view showing a state in which the dental implant driver according to an embodiment of the present invention is used, FIG. 2 is a perspective view of the dental implant driver according to an embodiment of the present invention, and FIG. 3 is a view showing the dental implant driver according to an embodiment of the present invention and an implant arranged together.

Referring to FIGS. 1 to 3, the dental implant driver 100 of the present invention is for aligning an implantation direction of an implant 30 implanted in an alveolar bone. A lower end thereof is coupled to the implant 30, and an upper end thereof is coupled to a rotating tool 20, such that a rotational force of the rotating tool 20 is transmitted to the implant 30.

Meanwhile, the implant 30 is inserted into the alveolar bone according to a rotational operation about an axis and serves as an artificial tooth root. For this purpose, the implant 30 has a substantially cylindrical shape and may be inserted into a bore hole previously formed in bone tissue of the alveolar bone at a position where a prosthesis is required. In a state in which the implant 30 is implanted in the alveolar bone, its implantation direction may be adjusted by the dental implant driver 100.

An upper recess to which an abutment for supporting a prosthesis is coupled may be formed in an upper end surface of the implant 30, and a thread may be formed on an outer surface thereof so that the implant 30 may be implanted in the alveolar bone. Further, the upper recess may include, from a lower side, an internal threaded portion 31 and a polygonal groove portion 32.

The internal threaded portion 31 is a portion disposed on the lower side of the upper recess, and a thread is formed on an inner circumferential surface thereof. A fastening screw for fixing an abutment may be coupled to the internal threaded portion 31, and, as will be described later, the support portion 110 of the dental implant driver 100 may be inserted thereinto and supported thereby.

The polygonal groove portion 32 is configured to prevent an abutment inserted into the upper recess from rotating about the axis. As will be described later, the insertion portion 120 of the dental implant driver 100 may be inserted into and fitted to the polygonal groove portion 32, whereby the rotational force of the driver may be transmitted to the implant 30. The polygonal groove portion 32 may preferably have a hexagonal shape, but is not limited thereto and may have various shapes.

Meanwhile, when the direction of the implant 30 is aligned, the dental implant driver 100 may be guided by a guide template 10.

The guide template 10 may be installed on a patient's gingiva and may have a guide hole 11 formed therein. At this time, the guide template 10 may have a direction confirmation groove 12 formed in an upper portion thereof. At least one such direction confirmation groove 12 may be formed around the guide hole 11. For example, the guide template 10 may be made of a 3D-printing material such as resin.

The guide hole 11 is configured to guide a movement path of at least one of a drill, an implant placement driver, and an implant direction alignment driver during an implant procedure, and may be formed in the shape of a hole penetrating a center of the guide template 10.

The direction confirmation groove 12 may be a groove used when the direction of the implant is aligned by using the dental implant driver 100. Specifically, the direction confirmation groove 12 may be recessed in advance at a position where one face of the polygonal groove portion 32 of the implant 30 is to be aligned. As will be described later, the practitioner may align the direction of the implant 30 by causing the identification element 141 of the dental implant driver 100 to coincide with the direction confirmation groove 12 of the guide template 10.

Referring to FIGS. 1 to 3, the dental implant driver 100 may include, from a lower side, a support portion 110, an insertion portion 120, an extension portion 130, a first direction recognition portion 140, a second direction recognition portion 150, and a mounting portion 160.

The support portion 110 is provided at a lower end of the dental implant driver 100, and, when coupled to the implant 30, may be disposed closer to the implant 30 than the insertion portion 120. The support portion 110 may be manufactured to have a diameter smaller than that of the internal threaded portion 31 of the implant 30. Accordingly, the dental implant driver 100 may easily enter the upper recess of the implant 30. Further, during rotation for alignment of the direction of the implant, the support portion 110 may be supported by the internal threaded portion 31, so that rotation of the dental implant driver 100 and alignment of the direction of the implant 30 may be smoothly performed.

The insertion portion 120 may be formed in a polygonal shape so as to be inserted into and fitted to the polygonal groove portion 32 of the implant 30, and may transmit the rotational force of the rotating tool 20 to the implant 30. According to one embodiment, the insertion portion 120 may be formed in a hexagonal shape having six faces.

The mounting portion 160 is provided at an upper end of the dental implant driver 100 and may be coupled to the rotating tool 20. The mounting portion 160 may have a firm fixing structure such that the rotating tool 20 does not separate therefrom. After mounting the rotating tool 20 on the mounting portion 160 of the dental implant driver 100, the practitioner may apply a force through the rotating tool 20 to finely adjust the direction of the implant 30.

The first direction recognition portion 140 is located above the insertion portion 120 and is configured for alignment of the implantation direction of the implant 30. The first direction recognition portion 140 may be formed in a substantially cylindrical shape so as to be guided by the guide hole 11 of the guide template 10. That is, an outer diameter of the first direction recognition portion 140 may correspond to an inner diameter of the guide hole 11 or may be formed to have a predetermined tolerance.

Meanwhile, one or more identification elements 141 having a direction aligned with at least one face of the polygonal insertion portion 120 may be formed on an outer circumferential surface of the first direction recognition portion 140 in order to align the implantation direction of the implant 30.

Since the identification element 141 has a direction aligned with at least one face of the insertion portion 120 that is inserted into and fitted to the polygonal groove portion 32 of the implant 30, the identification element 141 may perform a function of indicating the implantation direction of the implant 30.

That is, the practitioner may align the direction of the implant 30 by causing the identification element 141 of the dental implant driver 100 to coincide with the direction confirmation groove 12 of the guide template 10.

According to one embodiment, the one or more identification elements 141 may be formed spaced apart from one another at equal intervals along the outer circumference of the first direction recognition portion 140. In this case, the identification elements 141 may be formed in the same number as the number of faces of the polygonal insertion portion 120.

The identification elements 141 may be formed on the outer circumferential surface of the first direction recognition portion 140 in a recessed groove shape, and may be color-treated to facilitate recognition by the practitioner. As the identification elements 141 are formed in the recessed groove shape, guide elements 142 guided by the guide hole 11 of the guide template 10 may be formed between the identification elements 141.

Meanwhile, in aligning the direction of the implant 30, the practitioner generally causes the direction confirmation groove 12 of the guide template 10 and the identification element 141 of the dental implant driver 100 to coincide while viewing from the buccal direction. In the case of a molar, however, because it is difficult to secure a field of view, there may be a case in which it is difficult to confirm whether the direction confirmation groove 12 and the identification element 141 coincide.

To solve this problem, the dental implant driver 100 may include the second direction recognition portion 150, which is located above the first direction recognition portion 140 and assists in aligning the implantation direction of the implant 30.

Since the second direction recognition portion 150 is located above the first direction recognition portion 140 and is not inserted into the guide hole 11 of the guide template 10, when it is difficult to secure a field of view, the practitioner may supplementarily check and adjust the implantation direction of the implant 30 through the second direction recognition portion 150.

According to one embodiment, the second direction recognition portion 150 may include at least one angle 151 or face 152 having a direction aligned with the identification element 141.

At this time, the angle 151 or face 152 of the second direction recognition portion 150 may at least partially protrude in the radial direction beyond the first direction recognition portion 140.

When the direction of the angle 151 or face 152 of the second direction recognition portion 150 is configured to align with the direction of the identification element 141 as described above, the practitioner may easily align the direction of the implant 30 by causing the angle 151 or face 152 of the second direction recognition portion 150 to coincide with the direction confirmation groove 12 of the guide template 10.

The second direction recognition portion 150 may preferably be formed in an equilateral triangular shape. When the second direction recognition portion 150 is formed in an equilateral triangular shape, the angles 151 or faces 152 are arranged at intervals of 60 degrees, and thus it is easy to match the angle 151 or face 152 with the direction confirmation groove 12 of the guide template 10. Further, in a state in which the practitioner knows how much the implant 30 advances when the dental implant driver 100 rotates by 60 degrees, the practitioner may also finely adjust the insertion depth of the implant 30.

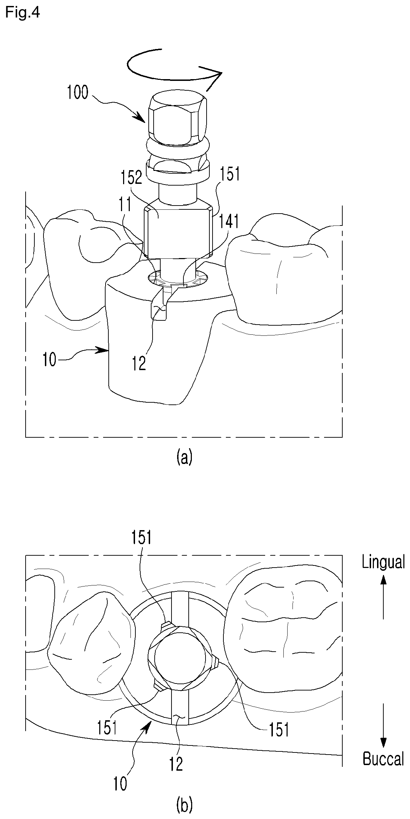

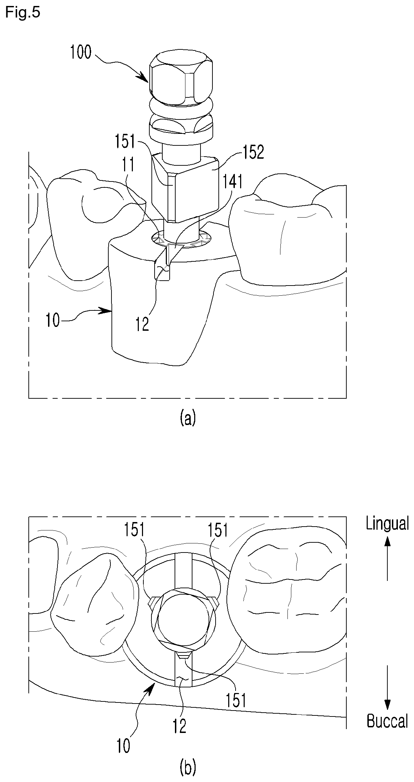

FIGS. 4A and 4B are a perspective view and a plan view, respectively, showing a state before alignment of the direction of an implant by using the dental implant driver according to an embodiment of the present invention, and FIGS. 5A and 5B are a perspective view and a plan view, respectively, showing a state after alignment of the direction of an implant by using the dental implant driver according to an embodiment of the present invention.

Referring to FIGS. 4 and 5, when it is difficult to secure a field of view, such as in a posterior tooth region, and it is therefore difficult to cause the identification element 141 of the first direction recognition portion 140 to coincide with the direction confirmation groove 12 of the guide template 10, the practitioner may easily align the direction of the implant 30 by rotating the second direction recognition portion 150, which has the equilateral triangular shape, such that the angle 151 or face 152 coincides with the direction confirmation groove 12.

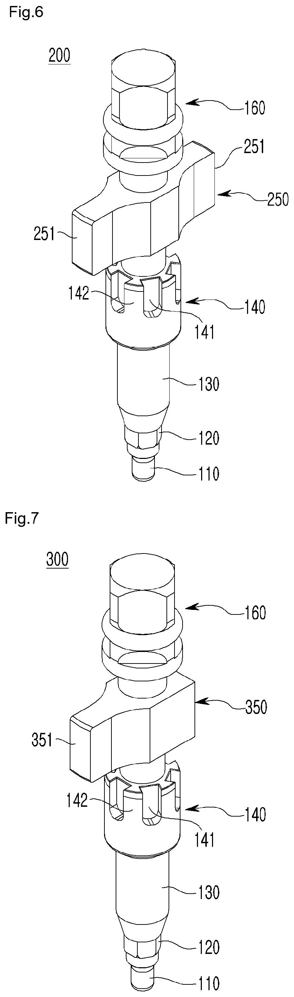

FIGS. 6 to 9 are views showing various embodiments of the dental implant driver of the present invention.

Referring to FIG. 6, the second direction recognition portion 250 of the implant driver 200 according to a second embodiment may be in a form in which blades 251 protrude toward both sides in the radial direction. An end of each blade 251 may have a form including a face or an angle, and the face or angle may have a direction aligned with the identification element 141 of the first direction recognition portion 140. That is, the practitioner may align the direction of the implant 30 by causing the face or angle of the blade 251 to coincide with the direction confirmation groove 12 of the guide template 10.

Referring to FIG. 7, the second direction recognition portion 350 of the implant driver 300 according to a third embodiment may be in a form in which a blade 351 protrudes toward one side in the radial direction. An end of the blade 351 may have a form including a face or an angle, and the face or angle may have a direction aligned with the identification element 141 of the first direction recognition portion 140. That is, the practitioner may align the direction of the implant 30 by causing the face or angle of the blade 351 to coincide with the direction confirmation groove 12 of the guide template 10.

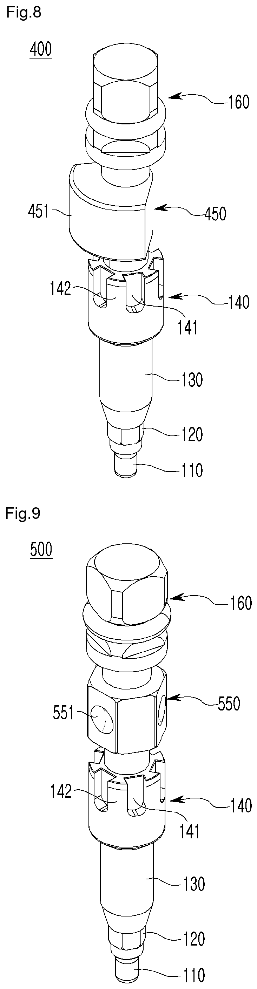

Referring to FIG. 8, the second direction recognition portion 450 of the implant driver 400 according to a fourth embodiment may be formed in a fan shape. The second direction recognition portion 450 having the fan shape may have a form including a face, preferably a curved surface 451, and a center of the curved surface 451 may have a direction aligned with the identification element 141 of the first direction recognition portion 140. That is, the practitioner may align the direction of the implant 30 by causing the center of the curved surface 451 of the second direction recognition portion 450 to coincide with the direction confirmation groove 12 of the guide template 10.

Referring to FIG. 9, the second direction recognition portion 550 of the implant driver 500 according to a fifth embodiment may be formed in a hexagonal shape. Each face of the second direction recognition portion 550 having the hexagonal shape may be arranged such that the direction thereof aligns with the identification element 141 of the first direction recognition portion 140. That is, the practitioner may align the direction of the implant 30 by causing one face 551 of the second direction recognition portion 550 to coincide with the direction confirmation groove 12 of the guide template 10.

As described above, since the dental implant driver of the present invention includes the first direction recognition portion and the second direction recognition portion, it is possible to easily determine the implantation direction of the implant implanted in the alveolar bone and intuitively align the implant in a desired direction, even in a situation in which it is difficult to secure a field of view during an implant procedure.

The foregoing description of the present invention is for illustrative purposes, and a person of ordinary skill in the art to which the present invention pertains will understand that the present invention can be easily modified into other specific forms without changing the technical spirit or essential features of the present invention. Therefore, it should be understood that the embodiments described above are illustrative in all respects and are not restrictive. For example, each component described as a single form may be implemented in a distributed form, and likewise, components described as distributed may also be implemented in a combined form.

The scope of the present invention is defined by the claims set forth below, and all changes or modified forms derived from the meaning and scope of the claims, and equivalents thereof, should be construed as being included in the scope of the present invention.

### Description of Reference Numerals

10: guide template
11: guide hole
12: direction confirmation groove
20: rotating tool
30: implant
31: internal threaded portion
32: polygonal groove portion
100: dental implant driver
110: support portion
120: insertion portion
130: extension portion
140: first direction recognition portion
141: identification element
142: guide element
150: second direction recognition portion
151: angle
152: face
160: mounting portion
200: dental implant driver according to the second embodiment
250: second direction recognition portion according to the second embodiment
300: dental implant driver according to the third embodiment
350: second direction recognition portion according to the third embodiment
400: dental implant driver according to the fourth embodiment
450: second direction recognition portion according to the fourth embodiment
500: dental implant driver according to the fifth embodiment
550: second direction recognition portion according to the fifth embodiment

## Claims

1. A dental implant driver for aligning an implantation direction of an implant implanted in an alveolar bone, the dental implant driver comprising:
a polygonal insertion portion provided at one end and configured to be inserted into and fitted to a polygonal groove portion of the implant;
a first direction recognition portion located above the insertion portion and having formed thereon one or more identification elements having a direction aligned with at least one face of the insertion portion for aligning the implantation direction of the implant; and
a second direction recognition portion located above the first direction recognition portion and having formed thereon an angle or a face having a direction aligned with at least one of the one or more identification elements for assisting in aligning the implantation direction of the implant.

2. The dental implant driver of claim 1, wherein the one or more identification elements are formed spaced apart from one another at equal intervals along a circumference of the first direction recognition portion.

3. The dental implant driver of claim 2, wherein the one or more identification elements are formed in a same number as a number of faces of the insertion portion.

4. The dental implant driver of claim 1, wherein the one or more identification elements are formed in a recessed groove shape.

5. The dental implant driver of claim 3, wherein the one or more identification elements are color-treated to facilitate identification.

6. The dental implant driver of claim 1, wherein the second direction recognition portion is formed in an equilateral triangular shape.

7. The dental implant driver of claim 1, wherein the second direction recognition portion is formed in a hexagonal shape.

8. The dental implant driver of claim 7, wherein a direction recognition groove is formed in at least one face of the second direction recognition portion.

9. The dental implant driver of claim 1, wherein the second direction recognition portion is formed in a fan shape.

10. The dental implant driver of claim 1, wherein the second direction recognition portion is formed with a blade protruding toward one side in a radial direction.

11. The dental implant driver of claim 1, wherein the second direction recognition portion is formed with two blades protruding toward both sides in a radial direction.
